# EUROPÄISCHE PATENTSCHRIFT

(11) **EP 1 280 603 B1**
(45) Veröffentlichungstag und Bekanntmachung des Hinweises auf die Patenterteilung: **18.08.2004**
(21) Anmeldenummer: 01945099.8
(22) Anmeldetag: 08.05.2001
(51) Int. Cl.: B01L 3/00, C12Q 1/68

(54) **VERFAHREN ZUR IDENTIFIZIERUNG, QUANTIFIZIERUNG UND VISUALISIERUNG VON MIKROORGANISMEN**
METHOD FOR THE IDENTIFICATION, QUANTIFICATION, AND VISUALISATION OF MICRO-ORGANISMS
PROCEDE D'IDENTIFICATION, DE QUANTIFICATION ET DE VISUALISATION DE MICRO-ORGANISMES

(30) Priorität: 08.05.2000 DE 10022304
(43) Veröffentlichungstag der Anmeldung: 05.02.2003
(73) Patentinhaber: Vermicon AG, 80992 München (DE)
(72) Erfinder: BEIMFOHR, Claudia, 80992 München (DE); SNAIDR, Jiri, 80992 München (DE)
(74) Vertreter: Maiwald Patentanwalts GmbH
(86) Internationale Anmeldenummer: PCT/EP2001/005226
(87) Internationale Veröffentlichungsnummer: WO 2001/085340

(56) Entgegenhaltungen:
- WO-A-92/07093
- WO-A-99/18234
- DE-A- 19 934 510
- US-A- 5 426 025
- "Hybridization guide" [Online] Juli 1996 (1996-07) , HYBAID LIMITED XP002198741 Gefunden im Internet: <URL: http://www.thermohybaid.com/cgi-bin/pg.exe ?0000000700000014000263cd000000b800003d08> [gefunden am 2002-05-13] das ganze Dokument
- SNAIDR JIRI ET AL: "Phylogenetic analysis and in situ identification of bacteria in activated sludge." APPLIED AND ENVIRONMENTAL MICROBIOLOGY, Bd. 63, Nr. 7, 1997, Seiten 2884-2896, XP002198740 ISSN: 0099-2240

## Beschreibung

Die Erfindung betrifft einen Kit zum Nachweis von Mikroorganismen in einer Probe, mit dem sowohl die Visualisierung als auch die Quantifizierung der Mikroorganismen möglich ist. Ferner betrifft die Erfindung ein Verfahren unter Verwendung dieses Kits.

Die Identifizierung von Mikroorganismen konnte viele Jahrzehnte nur nach vorhergehender Kultivierung der Mikroorganismen und damit einhergehender Vermehrung erfolgen. Diese Kultivierung erfolgt z.B. für Viren auf ihrem jeweiligen Wirtsorganismus, für Bakterien, Pilze und einzellige Algen in Nährmedien. Für die Erfassung der Anzahl lebensfähiger Mikroorganismen in einer bestimmten Probe wurden Medien bzw. Bedingungen gewählt, welche eine selektive Erfassung bestimmter Gruppen weitgehend ausschließen sollten. So wurden z.B. von bakteriellen Einzelkolonien Reinkulturen angelegt und diese aufgrund phänotypischer Merkmale, z.B. ihrer Morphologie und ihrer Stoffwechselprodukte, identifiziert. Die Identifizierung von Mikroorganismen nach vorheriger Kultivierung ist jedoch mit zwei entscheidenden Nachteilen verbunden. Erstens belegen Untersuchungen an den verschiedensten Umweltproben, daß nur 0,1 bis 14% aller Bakterien z.Z. kultivierbar und damit nachweisbar sind. Zweitens konnte bewiesen werden, daß bei der Kultivierung starke Populationsverschiebungen auftreten können, d.h. bestimmte Bakteriengruppen im Labor bevorzugt bzw. andere diskriminiert werden.

Dies bedeutet nicht nur, daß ein Großteil der Bakterien in zu untersuchenden Proben, z.B. Umweltproben, nicht erkannt werden kann, sondern auch, daß diejenigen Bakterien, welche identifiziert werden, meist ein verzerrtes Abbild der wahren Populationsstrukturen darstellen. Fehleinschätzungen der Populationsverhältnisse in bezug auf Identifizierung und Quantifizierung der Bakterien sind die Folge.

Anfang der neunziger Jahre wurde ein Verfahren zur in situ-Hybridisierung mit fluoreszenzmarkierten Oligonukleotidsonden entwickelt, das in vielen Umweltproben erfolgreich zum Einsatz kam (Amann et al. (1990) J. Bacteriol. 172:762). Dieses "FISH" (fluoreszierende in situ-Hybridisierung) genannte Verfahren beruht auf der Tatsache, daß die in jeder Zelle vorhandenen ribosomalen RNAs (rRNAs) hochkonservierte, d.h. wenig spezifische, und weniger konservierte, d.h. gattungs- und artspezifische Sequenzen umfassen. Schon Mitte der achtziger Jahre wurde gezeigt, daß die Sequenzen der 16S- und 23S-rRNA zur Identifizierung von Mikroorganismen genutzt werden können (Woese (1987) Microbiol. Reviews 51:221; De Long et al. (1989) Science 243:1360). Bei dem FISH-Verfahren werden fluoreszenzmarkierte Gensonden, deren Sequenzen zu einer bestimmten Region auf der ribosomalen Zielsequenz komplementär sind, in die Zelle geschleust. Die Sondenmoleküle sind in der Regel 16 bis 20 Basen lange, einzelsträngige Desoxyribonukleinsäurestücke und sind zu einem Zielbereich komplementär, der für eine bestimmte Bakterienart oder eine bakterielle Gattung spezifisch ist. Findet die fluoreszenzmarkierte Gensonde in einer Bakterienzelle ihre Zielsequenz, so bindet sie daran und die Zellen können aufgrund ihrer Fluoreszenz im Fluoreszenzmikroskop detektiert werden.

Es konnte gezeigt werden, daß durch die in situ-Hybridisierung mit fluoreszenzmarkierten Sonden nahezu 100% einer Bakteriengesamtpopulation detektiert werden können. Das Verfahren stellt daher bereits eine wesentliche Verbesserung gegenüber dem Stand der Technik dar, der die Detektion von maximal 14% der Bakterienpopulation einer Umweltprobe möglich machte. Darüber hinaus erlaubt das Verfahren der in situ-Hybridisierung mit fluoreszenzmarkierten Sonden, Aussagen über das Maß der Stoffwechselaktivität zu treffen, da die Anzahl der Ribosomen pro Zelle mit dieser Aktivität korreliert. Schließlich erlaubt das Verfahren, Bakterien direkt am Ort ihres Wirkens sichtbar zu machen, wodurch Wechselwirkungen zwischen verschiedenen Bakterienpopulation erkannt und analysiert werden können.

Innerhalb der letzten Jahre wurde die Technik der in situ-Hybridisierung mit fluoreszenzmarkierten Sonden für die verschiedensten Proben erprobt und erfolgreich angewandt. So konnten durch den Einsatz von Gensonden im Boden, in Protozoen, in Biofilmen, in der Luft, in Seen, in biologisch aktivierten Filtern und im Abwasser von Kläranlagen die jeweiligen Bakterienpopulationen untersucht und neuartige Bakterien identifiziert werden. Der Schwerpunkt lag hierbei in der Analyse der Bakterienpopulationen bei der Abwasserreinigung. Tropfkörper-, Raumfiltrations- und Belebtschlammanlagen wurden ebenso untersucht wie die Nachklärbecken und entsprechende Vorfluter (Snaidr et al. (1997) Appl. Environ. Microbiol. 63:2884). Durch die in situ-Hybridisierungstechnik konnte gezeigt werden, daß es bei der Erfassung der Belebtschlammflora durch Kultivierung zu einem Kultivierungsshift kommt (Wagner et al. (1993) Appl. Environ. Microbiol. 59:1520). Kultivierungsabhängige Verfahren liefern daher nur einen sehr verfälschten Einblick in die Zusammensetzung und Dynamik der mikrobiellen Biozönose. Durch diese Medium-abhängige Verzerrung der realen Verhältnisse innerhalb der Bakterienpopulation werden Bakterien, die im Belebtschlamm von Abwasserreinigungsanlagen nur eine untergeordnete Rolle spielen, aber den eingesetzten Kultivierungsbedingungen gut angepaßt sind, in ihrer Bedeutung dramatisch überschätzt. So konnte gezeigt werden, daß aufgrund eines solchen Kultivierungsartefakts die *Bakteriengattung Acinetobacter* bezüglich ihrer Rolle als biologischer Phosphatentferner in der Abwasserreinigung völlig falsch eingeschätzt wurde.

Obwohl die in situ-Hybridisierung mit den neu entwickelten fluoreszenzmarkierten Gensonden eine rasche und genaue Analyse von Bakterienpopulationen im Abwasser möglich macht, konnte sie sich in der Praxis noch nicht durchsetzen. Gründe hierfür sind der hohe Anschaffungspreis für die benötigten technischen Geräte wie Fluoreszenzmikroskope, der Bedarf an qualifizierten Kräften, welche für die Durchführung und Auswertung zur Verfügung stehen müssen, sowie die daraus resultierende geringe Anzahl möglicher Referenzmessungen. Weiterhin ist ein hoher Zeitaufwand für das Auszählen der detektierten Bakterienpopulationen (Quantifizierung) notwendig. Überdies erfordert das Auszählen hohe Erfahrungswerte, da zwischen echten (Sondenbindung hat tatsächlich stattgefunden) und falschen Signalen (Autofluoreszenz, keine Zellen) unterschieden werden muß.

Bei dem vorstehend beschriebenen Verfahren der FISH-Hybridisierung werden die Schritte der Zellfixierung, der Hybridisierung sowie der anschließende Waschschritt vor der Identifizierung der markierten Zellen jeweils in separaten Kammern durchgeführt. Die Hybridisierung findet dabei auf einem Objektträger statt, die Sonden dringen in die Zellen ein und bleiben an ihren Zielstellen haften. Nach einem Waschschritt zum Entfernen der nicht hybridisierten Nukleinsäuresondenmoleküle werden die markierten Zellen identifiziert.

Bei dem in der internationalen Patentanmeldung WO 99/18234 beschriebenen Verfahren zur qualitativen und quantitativen Analyse mikrobiologischer Populationen in einer Probe findet die Zellfixierung ebenfalls gesondert statt. Im Unterschied zu dem vorstehend beschriebenen Verfahren der FISH-Hybridisierung wird bei diesem Verfahren des Stand der Technik die Hybridisierung in einer Suspension durchgeführt. Die Nukleinsäuresonden dringen auch hier in die Zellen ein und bleiben an ihren Zielstellen haften. Nach einem Waschschritt zum Entfernen der nicht hybridisierten Nukleinsäuresonden werden die spezifisch gebundenen Sonden extrahiert und anschließend quantifiziert.

Nachteilig an dem vorstehend beschriebenen Verfahren ist, daß bei dem Abheben des Überstandes nach der Hybridisierung, üblicherweise durch Abpipettieren, auch ein Teil der markierten Zellen mitgenommen wird, was zu einer Verfälschung des Meßsignals führt. Aus dem selben Grund sind Zellbehandlungen, wie z.B. eine Ethanolbehandlung zur Dehydratisierung der Zellen oder eine Lysozymbehandlung für gram-positive Zellen, problematisch, da die hierfür erforderlichen Zentrifugationsschritte mit anschließender Abnahme des Überstandes häufig zu gravierenden Zellverlusten führen. Falls die Zellbehandlungen nicht durchgeführt werden, hat dies jedoch eine geringere Signalintensität zur Folge. Ferner sind dem Fachmann viele Bakterienarten (beispielsweise *Microthrix parvicella)* bekannt, bei denen ohne Vorbehandlung eine Penetration der Zellhülle nicht erfolgen kann. Schließlich können mit dem in der internationalen Patentanmeldung WO 99/18234 beschriebenen Verfahren manche Bakterienarten aufgrund geringer Signalintensitäten, bedingt durch die Hybridisierung in Suspension, nicht oder nur in unzureichendem Maße identifiziert und quantifiziert werden.

Aufgabe der vorliegenden Erfindung ist es daher, ein Verfahren bereitzustellen, mit dem eine schnelle und präzise Quantifizierung ohne Zellverluste möglich ist. Ferner ist es wünschenswert, einen Kit bereitzustellen, mit dem die Durchführung sowohl der Schritte der Identifizierung und Visualisierung als auch der Schritte der Identifizierung und Quantifizierung der markierten Zellen in einem Durchgang auf einfache Weise möglich ist.

Diese Aufgaben werden durch die Gegenstände der unabhängigen Ansprüche gelöst.

Vorteilhafte Ausgestaltungen sind in den Unteransprüchen beschrieben.

Erfindungsgemäß wird ein Verfahren zum Nachweis von Mikroorganismen in einer Probe mittels einer Nukleinsäuresonde bereitgestellt, wobei das Verfahren die folgenden Schritte umfaßt:
a) Fixieren der in einer Probe enthaltenden Mikroorganismen auf einem Träger;
b) Inkubieren der fixierten Mikroorganismen mit nachweisbaren Nukleinsäuresondenmolekülen auf einem Träger,
c) Entfernen der nicht hybridisierten Nukleinsäuresondenmoleküle;
d) Identifizieren der hybridisierten Nukleinsäuresondenmoleküle und/oder
e) Ablösen der hybridisierten Nukleinsäuresondenmoleküle und anschließendes Quantifizieren der abgelösten Nukleinsäuresondenmoleküle.

Dabei finden alle Reaktionen, d.h. das Inkubieren der fixierten Mikroorganismen, das Entfernen der nicht hybridisierten Nukleinsäuresondenmoleküle sowie, falls die markierten Zellen quantifiziert werden sollen, das Ablösen der hybridisierten Nukleinsäuresondenmoleküle in einem einzigen Gefäß statt. Bei einer bevorzugten Ausführungsform wird auch das Fixieren der in der Probe enthaltenen Mikroorganismen (Schritt a) in diesem Gefäß durchgeführt. Damit wird durch das erfindungsgemäße Verfahren erstmals ein Verfahren beschrieben, mit dem in ein und demselben Gefäß die Fixierung, Identifizierung und Visualisierung von Mikroorganismen und/oder die Fixierung, Identifizierung und Quantifizierung dieser Mikroorganismen möglich ist. Zellverluste durch unsachgemäßes Waschen, wie bei dem FISH-Verfahren beim Waschen der Objektträger, oder durch Abnehmen, wie bei dem in der internationalen Patentanmeldung WO 99/18234 beschriebenen Verfahren durch Abnehmen des Überstandes vom Pellet, sind bei dem erfindungsgemäßen Verfahren vollständig ausgeschlossen.

Bei einer bevorzugten Ausführungsform wird die zu untersuchende Probe in ein Gefäß (im folgenden auch als All-In-One (AIO)-Gefäß bezeichnet) hineinpipettiert. Das Gefäß mit vorzugsweise einem Filter als Träger wird in ein Auffanggefäß gesetzt. Die Probe wird abzentrifugiert und das Pellet wird direkt im AIO-Gefäß fixiert. Das AIO-Gefäß mit den fixierten Zellen kann entweder gelagert werden oder es kann unmittelbar weiterverwendet werden. Da die Zellen am Filter des AIO-Gefäßes haften, können sie nochmals für ein wirkungsvolles Eindringen der Sonden weiterbehandelt werden, beispielsweise durch Lysozymbehandlung bei grampositiven Zellen bzw. durch eine Ethanolbehandlung zur Dehydratisierung der Zellen. Die anschließende in situ-Hybridisierung findet ebenfalls im AIO-Gefäß statt. Dabei dringen spezifische, markierte Gensonden in die Zellen ein und heften sich an ihre Targetsequenzen. Anschließend wird ein Waschpuffer zum Entfernen der überschüssigen Sonden aufgetragen und wieder abzentrifugiert. Wird eine Visualisierung gewünscht, so wird der Filter entnommen und unter einem Fluoreszenzmikroskop betrachtet. Wird eine Quantifizierung gewünscht, so werden die auf dem Filter verbliebenen hybridisierten Zellen mit einem Ablösepuffer behandelt. Dabei kommt es zum Ablösen der spezifisch gebundenen Sonden. Wird der erneute Zentrifugationsschritt mit einem ungebrauchten Auffanggefäß durchgeführt, so kann das dort aufgefangene Filtrat anschließend gemessen werden. Dadurch können die in der Probe befindlichen Mikroorganismen identifiziert und quantifiziert werden.

Die beiden Abläufe Identifizierung und Visualisierung (Schritt d) sowie Identifizierung und Quantifizierung (Schritt e) können entweder alternativ oder in dieser Reihenfolge nacheinander durchgeführt werden. Falls gewünscht, kann auch die Fixierung im AIO-Gefäß durchgeführt werden. Die so fixierten Zellen können bis zu ihrer Verwendung im AIO-Gefäß gelagert werden.

Vorteile des erfindungsgemäßen Verfahrens gegenüber dem FISH-Verfahren sind zum einen die sehr leichte Handhabung, da bei diesem Verfahren alle Schritte in ein und demselben Reaktionsgefäß durchgeführt werden, und zum anderen die Wirtschaftlichkeit, da hier keine separaten Hybridisierungs- und Waschkammern benötigt werden.

Als Filter wird vorzugsweise ein Filter aus regenerierter Cellulose verwendet. Andere geeignete Filtermaterialien sind z.B. Cellulosenitrat, Celluloseacetat, Nitrocellulose, Mischester, Polycarbonat, Aluminiumoxid, Polyamid und dergleichen. Andere herkömmliche Filtermaterialien können zu einem sehr hohen Hintergrund bei der Visualisierung und vor allem bei der Quantifizierung führen, da bei dem Ablöseschritt oftmals Partikel aus dem Filter herausgelöst werden.

Vorteile des erfindungsgemäßen Verfahrens gegenüber dem in der internationalen Patentanmeldung WO 99/18234 beschriebenen Verfahren sind die sehr leichte Handhabung sowie die sehr schnelle Durchführbarkeit. Ferner treten insbesondere bei der Fixierung der in einer Probe enthaltenen Mikroorganismen auf einem Filter keine Zellverluste durch ein wie bei dem vorstehend genannten Verfahren notwendiges Abheben des Überstandes auf, wodurch präzise, standardisierbare Ergebnisse erhalten werden. Durch das Durchlaufen der Inkubations- bzw. Waschlösungen durch einen Filter wird vermieden, dass eventuell abgelöste Zellen bei einem sonst erforderlichen Abheben des Überstandes mit abgenommen werden und so Zellverluste entstehen. Darüber hinaus können auch Zellbehandlungen, wie eine Ethanolreihe oder eine Enzymbehandlung (z.B. mit Lysozym) für gram-positive Zellen vor der Hybridisierung ohne Zellverluste durchgeführt werden. Bei dem vorstehend genannten Verfahren führt der Verzicht auf eine derartige Vorbehandlung zu einer geringeren Flexibilität betreffend das Aufbrechen von Zellen sowie zu einer geringeren Signalintensität ohne Ethanolreihe. Falls bei dem vorstehend erwähnten Verfahren nicht auf eine derartige Vorbehandlung verzichtet wird, sind gravierende Zellverluste die Folge. Schließlich weist das erfindungsgemäße Verfahren gegenüber dem in WO 99/18234 beschriebenen Verfahren den Vorteil auf, daß eine Hybridisierung von Mikroorganismen, die zuvor auf einem Träger fixiert wurden, zu wesentlich stärkeren Signalintensitäten führt, als eine wie dort beschriebene Hybridierung in Suspension.

Bei einer weiteren Ausfuhrungsform der vorliegenden Erfindung kann das erfindungsgemäße Verfahren auch im Mikrotiterformat und damit automatisierbar angewandt werden. Hierbei wird ein entsprechend kleiner Filter auf die Mikrotiterplatte gesetzt, dem erfindungsgemäßen AIO-Gefäß entspricht in diesem Fall das Mikrotiterwell mit dem Filter als Boden. Die Mikrotiterplatte wird abzentrifugiert, oder die Flüssigkeiten werden mit Hilfe einer Vakuumfiltrationsapparatur filtriert. Die extrahierten markierten Nukleinsäuresonden befinden sich dann im Mikrotiterwell.

Erfindungsgemäß wird bei einem weiteren Aspekt der vorliegenden Erfindung ein Kit zur Durchführung des Verfahrens zum Nachweis von Mikroorganismen in einer Probe bereitgestellt. Der Inhalt eines solchen Kits richtet sich im wesentlichen nach der Natur des nachzuweisenden Mikroorganismus. Er umfaßt als wichtigsten Bestandteil eine für den jeweils nachzuweisenden Mikroorganismus spezifische Nukleinsäuresonde sowie bevorzugt eine weitere Nukleinsäuresonde, mit der eine Negativkontrolle durchgeführt werden kann. Darüber hinaus umfaßt er vorzugsweise einen Hybridisierungspuffer und gegebenenfalls einen Lysepuffer. Die Wahl des Hybridisierungspuffers hängt in erster Linie von der Länge der verwendeten Nukleinsäuresonden ab. So müssen, wie dem Fachmann bekannt ist, für die Hybridisierung einer Nukleinsäuresonde von 15 Nukleotiden Länge weniger stringente Bedingungen gewählt werden als für die Hybridisierung einer Sonde von 75 Nukleotiden Länge. Beispiele für Hybridisierungsbedingungen sind z. B. in *Stahl* & Amann (1991) in Stackebrandt u. Goodfellow (Hrsg.), Nucleic Acid Techniques in Bacterial Systematics; John Wiley & Sons Ltd., Chichester, UK, angegeben.

Die Zusammensetzung des Lysepuffers ist ebenfalls vom jeweiligen Mikroorganismus abhängig. So sind zur Lyse von Virushüllen, Zellwänden grampositiver oder gram-negativer Bakterien und Zellmembranen von Hefe oder Algen jeweils leicht unterschiedliche Bedingungen erforderlich, die ohne weiteres in der Literatur festgestellt werden können.

Erfindungsgemäß wird somit ein Kit bereitgestellt, mit dem das vorstehend beschriebene erfindungsgemäße Verfahren durchgeführt werden kann. Der erfindungsgemäße Kit umfasst in einer bevorzugten Ausführungsform mindestens einen Hybridisierungspuffer, mindestens eine Nukleinsäuresonde zum spezifischen Nachweis eines Mikroorganismus, mindestens eine Nukleinsäuresonde zum Durchführen einer Negativkontrolle, ein Gefäß (AIO-Gefäß) und gegebenenfalls ein Auffanggefäß, in welches das AIO-Gefäß eingebracht werden kann, wobei in dem AIO-Gefäß die folgenden Schritte durchführbar sind:
a) Fixieren der in der Probe enthaltenen Mikroorganismen auf einem Träger, insbesondere einem Filter, besonders bevorzugt einem Filter aus regenerierter Cellulose;
b) Inkubieren der fixierten Mikroorganismen mit nachweisbaren Nukleinsäuresondenmolekülen auf einem Träger;
c) Entfernen nicht hybridisierter Nukleinsäuresondenmoleküle; und
d) Ablösen der hybridisierten Nukleinsäuresondenmoleküle.

Der Träger, vorzugsweise der Filter, ist in einer bevorzugten Ausführungsform des AIO-Gefäßes von dem Gefäß abnehmbar, so daß die Visualisierung der markierten Zellen, beispielsweise unter einem Epifluoreszenzmikroskop, leicht durchführbar ist. Ferner ist das AIO-Gefäß und/oder das Auffanggefäß vorzugsweise verschließbar, um eine sichere Zentrifugation zu ermöglichen.

In einer bevorzugten Ausführungsform enthält der erfindungsgemäße Kit spezifische Sonden zum Nachweis von filamentösen Bakterien aus der Gruppe der grünen, nicht-schwefelhaltigen Bakterien, von *Nostocoida limicola-*ähnlichen Bakterien und von filamentösen *Rhodobacter sphaeroides*-ähnlichen Bakterien.

Im Rahmen der vorliegenden Erfindung soll unter "Fixieren" von Mikroorganismen eine Behandlung verstanden werden, mit der die den jeweiligen Mikroorganismus umgebenden Hüllen so durchlässig gemacht werden sollen, daß die Nukleinsäuresonde mit der gegebenenfalls kovalent verbundenen Markierung durch die Hülle penetrieren kann, um so die Zielsequenzen im Zellinneren erreichen zu können. Bei der Hülle kann es sich z.B. um die ein Virus umgebende Lipidhülle, um die Zellwand eines Bakteriums oder die Zellmembran eines einzelligen Mikroorganismus handeln. Zur Fixierung wird üblicherweise eine geringprozentige Paraformaldehydlösung verwendet. Sollte mit einer Paraformaldehydlösung im Einzelfall die einen Mikroorganismus umgebende Schutzhülle nicht penetrierbar gemacht werden können, so sind dem Fachmann ausreichend weitere Maßnahmen bekannt, die zu demselben Ergebnis führen. Dazu zählen beispielsweise Formaldehyd, Methanol, Mischungen von Alkoholen mit Paraformaldehyd, enzymatische Behandlungen, Ultraschallbehandlung etc. Bei einer bevorzugten Ausführungsform der vorliegenden Erfindung wird die Fixierung mit Ethanol, besonders bevorzugt mit 50%igem Ethanol durchgeführt.

Bei einer Nukleinsäuresonde im Sinne der Erfindung kann es sich um eine DNAoder RNA-Sonde handeln, die in der Regel zwischen 12 und 1000 Nukleotide umfassen wird, bevorzugt zwischen 12 und 100 oder 15 und 50, besonders bevorzugt zwischen 17 und 25 Nukleotide. Die Nukleinsäuresonde ist so ausgewählt, daß es eine zu ihr komplementäre Sequenz im nachzuweisenden Mikroorganismus bzw. in der Gruppe nachzuweisender Mikroorganismen gibt. Komplementarität sollte bei einer Sonde von nur ca. 15 Nukleotiden möglichst über die gesamte Sequenz gegeben sein, bei Oligonukleotiden mit mehr als 15 Nukleotiden sind ein bis mehrere Fehlpaarungsstellen erlaubt. Es muß jedoch gewährleistet sein, daß das Nukleinsäuresondenmolekül mit moderaten und/oder stringenten Hybridisierungsbedingungen tatsächlich mit der Zielsequenz hybridisiert. Moderate Bedingungen im Sinne der Erfindung sind z.B. weniger als 20% Formamid in einem Hybridisierungspuffer wie er in Beispiel 1 beschrieben ist. Stringente Bedingungen im Sinne der Erfindung sind beispielsweise 20 - 80% Formamid in dem in Beispiel 1 beschriebenen Puffer.

Die Dauer der Hybridisierung beträgt üblicherweise zwischen 10 Minuten und 12 Stunden; bevorzugt erfolgt die Hybridisierung für etwa 2 Stunden. Die Hybridisierungstemperatur beträgt bevorzugt zwischen 44 °C und 48 °C, besonders bevorzugt 46 °C, wobei der Parameter der Hybridisierungstemperatur, wie auch die Konzentration an Salzen und Detergentien in der Hybridisierungslösung in Abhängigkeit von der Sonde bzw. den Sonden, insbesondere deren Länge(n) und dem Grad der Komplementarität mit der Targetsequenz in der nachzuweisenden Zelle optimiert werden kann. Der Fachmann ist mit hier einschlägigen Berechnungen vertraut.

Im Rahmen des erfindungsgemäßen Verfahrens hat eine typische Hybridisierungslösung eine Salzkonzentration von 0,1 mol/l bis 1,5 mol/l, bevorzugt von 0,9 mol/l, wobei es sich bei dem Salz vorzugsweise um Natriumchlorid handelt. Weiter umfaßt der Hybridisierungspuffer üblicherweise ein Detergens, wie z.B. Natriumdodecylsulfat (SDS), in einer Konzentration von 0,001-0,1%, bevorzugt in einer Konzentration von 0,01 %, und Tris/HCl in einem Konzentrationsbereich von 0,001-0,1 mol/l, vorzugsweise in einer Konzentration von 0,02 mol/l. Der pH-Wert von Tris/HCl beträgt üblicherweise zwischen 6 und 10, wobei ein pH von ca. 8,0 bevorzugt ist. Wie oben erwähnt, kann die Hybridisierungslösung des weiteren zwischen 0% und 80% Formamid enthalten, je nachdem welcher Grad von Stringenz erwünscht ist bzw. benötigt wird.

Die Nukleinsäuresonde sollte im Hybridisierungspuffer wenn möglich in einer Menge von 15 ng bis 1000 ng anwesend sein, wobei diese Menge in einem Volumen an Hybridisierungslösung zwischen 8 µl und 100 µl, vorzugsweise in 80 µl, enthalten ist. Besonders bevorzugt beträgt die Sondenkonzentration 125 ng/80 µl Hybridisierungslösung.

Nach erfolgter Hybridisierung sollten die nicht hybridisierten und überschüssigen Sondenmoleküle entfernt werden, was üblicherweise mittels einer herkömmlichen Waschlösung bzw. eines herkömmlichen Waschpuffers erfolgt. Dieser Waschpuffer enthält gewöhnlicherweise 0,001-0,1% eines Detergens wie SDS, wobei eine Konzentration von 0,01% bevorzugt wird, und Tris/HCl in einer Konzentration von 0,001-0,1 mol/l, bevorzugt 0,02 mol/l, wobei der pH-Wert von Tris/HCl im Bereich von 6,0 bis 10,0, vorzugsweise bei 8,0 liegt. Weiter enthält der Waschpuffer üblicherweise NaCl, wobei die Konzentration je nach benötigter Stringenz von 0,003 mol/l bis 0,9mol/l, bevorzugt von 0,01 mol/l bis 0,9 mol/l, beträgt. Zusätzlich kann die Waschlösung EDTA enthalten, wobei die Konzentration vorzugsweise 0,005 mol/l beträgt.

Das "Abwaschen" der nicht gebundenen Sondenmoleküle erfolgt üblicherweise bei einer Temperatur im Bereich von 44 °C bis 52 °C, bevorzugt von 46°C bis 50°C und besonders bevorzugt bei 48 °C für eine Dauer von 10-40 Minuten, vorzugsweise für 20 Minuten.

Nach erfolgter in situ-Hybridisierung und anschließender Entfernung der nichthybridisierten Nukleinsäuresondenmoleküle mittels des oben beschriebenen Waschschrittes erfolgt die Abtrennung der hybridisierten Sondenmoleküle für die Detektion und, falls erwünscht, Quantifizierung der hybridisierten Sondenmoleküle. Für diesen Extraktionsschritt sind solche Extraktionsmittel geeignet, die bei einer geeigneten Temperatur eine Denaturierung der Sonde von der Targetsequenz gewährleisten, ohne die Sondemoleküle in nennenswertem Ausmaß zu beschädigen. Bevorzugt wird hierfür als Ablöselösung bzw. Ablösepuffer Wasser, also H₂O_{dest./bidest.,} oder schwach gepuffertes Wasser, also bspw. Tris/HCl, im Konzentrationsbereich von 0,001 mol/l bis 1,0 mol/l, besonders bevorzugt in einer Konzentration von 0,01 mol/l, verwendet, wobei der pH von Tris/HCl von 7,0 bis 11,0, vorzugsweise 9,0 beträgt. Weiter sind im Rahmen dieser Erfindung auch DMSO (Dimethylsulfoxid) und 1 x SSC, pH 10,0 (+/- 2,0) als Extraktionsmittel geeignet, wobei 1 x SSC geeigneterweise durch Verdünnung einer 20 x SSC-Stammlösung (175,2 g NaCl, 88,2 g Natriumcitrat, auffüllen mit Wasser auf 1 Liter) zubereitet wird.

Die Ablösung der Sondenmoleküle erfolgt üblicherweise für eine Dauer von 5 bis 30 Minuten, bevorzugt für 15 Minuten. Dabei erfolgt die Sondenextraktion in einem Temperaturbereich von 50-100 °C, vorzugsweise bei etwa 80 °C. In jedem Fall sollte versucht werden, die Extraktion bei einer Temperatur durchzuführen, die eine effektive, aber gleichzeitig für die Sondenmoleküle schonende Abtrennung gewährleistet. Da die Sonden durch die Extraktionsbehandlung umso weniger beeinträchtigt werden, je geringer die Temperatur ist, wird eine Temperatur < 100 °C, insbesondere < 90 °C bevorzugt.

Als Extraktionsmittel werden insbesondere H₂O_{bidest} oder 0,01 mol/l Tris/HCl gegenüber Formamid, das im Stand der Technik in der Regel als Extraktionsmittel empfohlen wird, bevorzugt. Durch den Verzicht von Formamid im Extraktionsschritt erzielt man nicht nur eine schonendere Behandlung für die markierten Sonden, es ergeben sich zusätzlich auch Vorteile hinsichtlich des Gefahrenpotentials, da es sich bei Formamid um eine Substanz der Giftklasse 3 handelt. Darüber hinaus ist die Verwendung von Formamid im Vergleich zu Wasser oder schwach gepuffertem Wasser wesentlich kostenintensiver.

Die Auswahl der jeweiligen Nukleinsäuresonde erfolgt in Abhängigkeit vom nachzuweisenden Mikroorganismus: Sollen beispielsweise nur Mikroorganismen der Gattung *Streptococcus salivarius* nachgewiesen werden, nicht jedoch Mikroorganismen der Gattung *Streptococcus thermophilus, so* wird der Fachmann eine geeignete Sequenz auswählen, die in *Streptococcus salivarius* auftritt, in *Streptococcus thermophilus* dagegen nicht. Typischerweise werden diese Sequenzen aus der 16S-oder 23S-rRNA ausgewählt. Ist dagegen erwünscht, alle Bakterien der Gattung *Streptococcus* zu erfassen, wird eine Sequenz ausgewählt werden, die *Streptococcus salivarius* und *Streptococcus thermophilus* sowie weiteren Spezies der Gattung *Streptococcus* gemeinsam ist. Für solche Sequenzen sind in der Literatur bereits viele Beispiele veröffentlicht, siehe z.B. Beimfohr et al. (1993) System Appl. Microbiol. 16:450. Die Nukleinsäuresonde kann dabei komplementär zu einer chromosomalen oder episomalen DNA sein, aber auch zu einer mRNA oder rRNA des nachzuweisenden Mikroorganismus. Dabei ist es bevorzugt, Nukleinsäuresonden zu wählen, die in einem Bereich komplementär sind, der in einer Kopienzahl von mehr als 1 im jeweilig nachzuweisenden Mikroorganismus vorliegt. Die nachzuweisende Sequenz liegt bevorzugt 500 - 100.000 mal pro Zelle vor, besonders bevorzugt 1000 - 50.000 mal. Dies ist ein weiterer Grund, warum die Nukleinsäuresonde besonders bevorzugt komplementär zu einer rRNA ist: die ribosomalen RNAs sind Bestandteile der Ribosomen, die, da sie die Proteinsynthesemoleküle darstellen, in jeder aktiven Zelle vieltausendfach vorhanden sind.

Erfindungsgemäß wird die Nukleinsäuresonde mit dem im oben genannten Sinne fixierten Mikroorganismus inkubiert, um so ein Eindringen der Nukleinsäuresondenmoleküle in den Mikroorganismus und die Hybridisierung von Nukleinsäuresondenmolekülen mit den Nukleinsäuren des Mikroorganismus zu erlauben. Anschließend werden nicht hybridisierte Nukleinsäuresondenmoleküle durch übliche Waschschritte entfernt. Falls eine Quantifizierung erwünscht ist, werden im Gegensatz zum herkömmlichen FISH-Verfahren nunmehr jedoch nicht die hybridisierten Nukleinsäuresondenmoleküle in situ, also im jeweiligen Mikroorganismus, belassen, sondern von der nachzuweisenden Nukleinsäure wiederum abgelöst, von zellulären Bestandteilen getrennt, detektiert und quantifiziert.

Voraussetzung sowohl für die Identifizierung als auch für die Quantifizierung ist, daß das erfindungsgemäß verwendete Nukleinsäuresondenmolekül nachweisbar ist. Diese Nachweisbarkeit kann z.B. durch kovalente Verbindung des Nukleinsäuresondenmoleküls mit einem detektierbaren Marker sichergestellt werden. Als detektierbare Marker werden üblicherweise fluoreszierende Gruppen, z.B. Cy-2, Cy-3 oder Cy-5, FITC, CT, TRITC oder Fluos-Prime verwendet, die dem Fachmann alle wohlbekannt sind; der Vollständigkeit halber sind einige Marker, ihre Eigenschaften und Bezugsquellen in der folgenden Tabelle 1 angegeben.

**TABELLE 1**

| | |
|---|---|
| FLUOS: | 5,(6)-Carboxyfluorescein-N-hydroxysuccinimedester (Boehringer Mannheim, Mannheim, Deutschland); ε = 7,50 x 104 mol⁻¹ 1⁻¹, Absₘₐₓ bei 494 nm; Emₘₐₓ bei 518nm, MG = 473. |
| TRITC: | Tetramethylrhodamin-5,6 isothiocyanat (Isomer G. Molecular Probes Inc., Eugene, USA, Lambda, Graz, AT); ε = 1,07 x 105 mol⁻¹ 1⁻¹, Absₘₐₓ bei 537 nm; Emₘₐₓ bei 566 nm, MG = 479. |
| CT: | 5,(6)-Carboxytetramethylrhodamin-N-hydroxysuccinimidester (Molecular Probes Inc., Eugene, USA); ε = 0,87 x 105 mol⁻¹ 1⁻¹, Absₘₐₓ bei 537 nm; Emₘₐₓ bei 566 nm. |
| CY-3: | NHS-Ester von Cy5.18 (Biological Detection Systems, Pittsburgh, USA); (Amersham Life Sciences, Inc., Arlington Heights, USA); ε = 1,5 x 105 mol⁻¹ 1⁻¹, Absₘₐₓ bei 532 nm; Emₘₐₓ bei 565 nm. MG = 765,95. |
| CY-5: | NHS-Ester von Cy5.18 (Biological Detection Systems, Pittsburgh, USA); (Amersham Life Sciences, Inc., Arlington Heights, USA); ε = > 2 x 105 mol⁻¹ 1⁻¹, Absₘₐₓ bei 650 nm; Emₘₐₓ bei 667 nm. MG = 791,99. |

Alternativ werden chemolumineszierende Gruppen oder radioaktive Markierungen, z.B. ³⁵S, ³²P, ³³P, ¹²⁵J, verwendet. Nachweisbarkeit kann aber auch gegeben sein durch Kopplung des Nukleinsäuresondenmoleküls mit einem enzymatisch aktiven Molekül, beispielsweise alkalischer Phosphatase, saurer Phosphatase, Peroxidase, Meerrettichperoxidase, β-D-Galaktosidase oder Glukoseoxidase. Für jedes dieser Enzyme ist eine Reihe von Chromogenen bekannt, die anstelle des natürlichen Substrats umgesetzt werden können, und entweder zu farbigen oder zu fluoreszierenden Produkten umgesetzt werden können. Beispiele für solche Chromogene sind in der nachfolgenden Tabelle 2 angegeben.

**TABELLE 2**

| Enzyme | Chromogen |
|---|---|
| 1. Alkalische Phosphatase und saure Phosphatase | 4-Methylumbelliferylphosphat (*), Bis(4-Methyiumbelliferylphosphat), (*) 3-O-Methylfluoreszein, Flavon-3-Diphosphattriammoniumsalz (*), p-Nitrophenylphosphatdinatriumsalz |
| 2. Peroxidase | Tyraminhydrochlorid (*), 3-(p-Hydroxyphenyl)-Propionsäure (*), p-Hydroxyphenethylalkohol (*),2,2'-Azino-di-3-ethylbenzthiazolin-sulfonsäure (ABTS), ortho-Phenylendiamindihydrochlorid, o-Dianisidin, 5-Aminosalicylsäure, p-Ucresol (*), 3,3'-dimethyloxybenzidin, 3-Methyl-2-benzothiazolinhydrazon, Tetramethylbenzidin |
| 3. Meerrettichperoxidase | H₂O₂ + Diammoniumbenzidin H₂O₂ + Tetramethylbenzidin |
| 4. β-D-Galaktosidase | o-Nitrophenyl-β-D-galaktopyranosid, 4-Methylumbelliferyl-β-D-galaktosid |
| 5. Glukoseoxidase | ABTS, Glukose und Thiazolylblau |

| | |
|---|---|
| * Fluoreszenz | |

Schließlich ist es möglich, die Nukleinsäuresondenmoleküle so zu gestalten, daß an ihrem 5'- oder 3'-Ende eine weitere zur Hybridisierung geeignete Nukleinsäuresequenz vorhanden ist. Diese Nukleinsäuresequenz umfaßt wiederum ca. 15 bis 1000, bevorzugt 15 bis 50 Nukleotide. Dieser zweite Nukleinsäurebereich kann wiederum von Oligonukleotidsonden erkannt werden, die durch eines der oben erwähnten Mittel nachweisbar sind.

Eine weitere Möglichkeit besteht in der Kopplung der nachweisbaren Nukleinsäuresondenmoleküle mit einem Hapten. Nach Ablösung der Nukleinsäuresondenmoleküle von der Zielnukleinsäure können die nunmehr separat vorliegenden Nukleinsäuresondenmoleküle mit das Hapten erkennenden nachweisbaren Antikörpern in Kontakt gebracht werden. Ein bekanntes Beispiel für ein solches Hapten ist Digoxigenin oder seine Derivate. Dem Fachmann sind über die angegebenen Beispiele hinaus viele weitere Möglichkeiten bekannt, ein zur Hybridisierung verwendetes Oligonukleotid zu detektieren und zu quantifizieren.

Die Vielzahl möglicher Markierungen ermöglicht auch den gleichzeitigen Nachweis von zwei oder mehr verschiedenen, sich überlappenden oder sich nicht überlappenden Populationen. So kann z.B. durch Verwendung von zwei verschiedenen Fluoreszenzmarkern Streptococcus *salivarius* neben *Streptococcus thermophilus,* oder *Streptococcus salivari*us neben der Streptococcen-Gesamtpopulation nachgewiesen werden.

Der mittels des erfindungsgemäßen Verfahrens nachzuweisende Mikroorganismus kann ein prokaryontischer oder eukaryontischer Mikroorganismus sein. In den meisten Fällen wird es erwünscht sein, einzellige Mikroorganismen nachzuweisen. Relevante Mikroorganismen sind dabei vor allem Hefen, Bakterien, Algen oder Pilze.

In einer besonders bevorzugten Ausführungsform der vorliegenden Erfindung handelt es sich bei dem Mikroorganismus um filamentöse Bakterien aus der Gruppe der grünen, nicht-schwefelhaltigen Bakterien, *Nostocoida limicola-*ähnliche Bakterien und filamentöse *Rhodobacter sphaeroides-*ähnlichen Bakterien.

Das erfindungsgemäße Verfahren kann vielfältig angewendet werden. So ist es z.B. geeignet, Umweltproben auf das Vorhandensein bestimmter Mikroorganismen zu untersuchen. Diese Umweltproben können aus dem Wasser, aus dem Boden oder aus der Luft entnommen sein. Für den Nachweis von bestimmten Bakterien in Umweltproben ist normalerweise keinerlei vorausgehende Kultivierung notwendig.

Ein weiteres Anwendungsgebiet für das erfindungsgemäße Verfahren ist die Kontrolle von Lebensmittelproben. In bevorzugten Ausführungsformen werden die Lebensmittelproben aus Milch oder Milchprodukten (Joghurt, Quark, Käse, Butter, Buttermilch), Trinkwasser, Getränken (Säfte, Limonade, Bier), Backwaren oder Fleischwaren entnommen. Für den Nachweis von Mikroorganismen in Lebensmitteln kann u. U. eine vorherige Kultivierung erwünscht oder sogar vorgeschrieben sein. So ist es notwendig, z. B. für den Nachweis von einer einzigen Salmonelle in 25 ml Milch, diese eine Zeitlang zu kultivieren, um anschließend auch mit statistischer Zuverlässigkeit eine oder mehrere Salmonellen im Probenvolumen zu haben.

Das erfindungsgemäße Verfahren kann weiter zur Untersuchung medizinischer Proben eingesetzt werden. Dabei ist es sowohl für die Untersuchung von Gewebeproben, z.B. Biopsiematerial aus der Lunge, Tumor- oder entzündlichem Gewebe, aus Sekreten wie Schweiß, Speichel, Sperma und Ausfluß aus der Nase, Harnröhre oder Vagina sowie für Stuhl- und Urinuntersuchungen geeignet.

Ein weiteres wichtiges Anwendungsgebiet für das vorliegende Verfahren ist die Untersuchung von Abwässern, z.B. Belebtschlamm, Faulschlamm oder anaerobem Schlamm. Darüber hinaus ist es geeignet, Biofilme in industriellen Anlagen zu analysieren, sowie auch sich natürlicherweise bildende Biofilme oder bei der Abwasserreinigung bildende Biofilme zu untersuchen. Schließlich ist es auch zur Untersuchung und Qualitätskontrolle pharmazeutischer und kosmetischer Produkte, z.B. von Salben, Cremes, Tinkturen, Säften etc. geeignet.

Obwohl die Erfindung im wesentlichen mit Bezug auf fluoreszenzmarkierte Sondenmoleküle beschrieben worden ist, versteht es sich von selbst, daß die genannten Vorteile bei Verwendung anderer Marker ebenfalls gegeben sind.

Die folgenden Beispiele und Abbildungen dienen der Erläuterung der Erfindung und sollen nicht in einschränkender Weise ausgelegt werden.

### Beispiel 1: Identifizierung und Visualisierung von Nostocoida limicola-ähnlichen fadenförmigen Bakterien in Belebtschlammproben

### Schritt A: Zellfixierung

Zur Zellfixierung werden 2 ml-Reaktionsgefäße sowie eine Tischzentrifuge (14.000 Umdrehungen/Minute) benötigt.

Zur Paraformaldehyd-Zellfixierung wurden folgende Lösungen verwendet:
- PBS-Stammlösung (NaₓPO₄)
   200 mmol/l NaH₂PO₄
   200 mmol/l Na₂HPO₄ (pH 7,2-7,4)
- 3 x PBS-Lösung
   390 mmol/l NaCl
   30 mmol/l NaₓPO₄ (PBS-Stammlösung) (pH 7,2-7,4)
- 1 x PBS-Lösung
   130 mmol/l NaCl
   10 mmol/l NaₓPO₄ (PBS-Stammlösung) (pH 7,2-7,4)
- 96%-ige Ethanollösung.

Die Zellfixierung wurde auf folgende Weise durchgeführt:
1. Zugabe von einem Teil 96%-iger Ethanollösung zu einem Teil frisch gezogener Belebtschlammprobe (z.B. 10 ml 96%-ige Ethanollösung zu 10 ml Belebtschlamm).
2. Inkubation für 1 Stunde bei 4 °C.

Auf diese Weise wurde eine fixierte Belebtschlammprobe erhalten.

### Schritt B: Hybridisierung

Für die in situ-Hybridisierung werden benötigt:
- Tischzentrifuge (14.000 Umdrehungen pro Minute)
- Fluorometer
- 1 Heizblock und 1 Wasserbad, oder 2 Heizblöcke
- Hybridisierungsofen
- All-In-One-Gefäße mit Auffanggefäß
- Formamid (Merck, Darmstadt, Deutschland)
- Sonden-Arbeitslösungen zu je 50 ng/ µl:
- Ablösepuffer: 0,01 mol/l Tris/HCl (pH 9,0)
- Hybridisierungspuffer (2 ml mit 20 % Formamidgehalt):

| | |
|---|---|
| 5 mol/l NaCl | 360 µl |
| 1 mol/l Tris/HCl pH 8,0 | 40 µl |
| Formamid | 400 µl |
| 10 % (m/v) SDS | 2 µl |
| H₂O _{bidest} | 1200 µl |

- Waschpuffer (2 ml):

| | |
|---|---|
| 5 mol/l NaCl | 86 µl |
| 1 mol/l Tris/HCl pH 8,0 | 40 µl |
| 0,5mol/l EDTA pH 8,0 | 20 µl |
| 10 % (m/v) SDS | 2 µl |
| H₂O _{bidest} | 1850 µl |

### Durchführung:

5 bis 500 µl der fixierten Zellsuspension wurden in die Mitte eines AIO-Gefäßes pipettiert und bei 14.000 Umdrehungen pro Minute für 6 Minuten abzentrifugiert. Das Filtrat wurde verworfen. Auf das Pellet wurden 500 µl einer 50 %-igen Ethanollösung pipettiert und 2 Minuten inkubiert. Nach dem neuerlichen Abzentrifugieren (14.000 Umdrehungen pro Minute, 2 Minuten) wurde das Pellet für 2 Minuten mit 500 µl einer 70 %-igen Ethanollösung inkubiert und erneut einer Zentrifugation unterzogen (14.000 Umdrehungen pro Minute, 2 Minuten). Danach wurden 500 µl einer 96 %-igen Ethanollösung auf das Pellet pipettiert und für 2 Minuten inkubiert. Nach einem anschließenden Zentrifugationsschritt (14.000 Umdrehungen pro Minute, 5 Minuten) wurde das AIO-Gefäß für 5 Minuten bei 80 °C auf einem Heizblock inkubiert. Nach der Zugabe von 20 µl des auf 46 °C vorgewärmten Hybridisierungspuffers wurden 2 µl jeder Oligonukleotidsonde (Konzentration 50 ng/ µl) hinzugefügt. Anschließend wurde der Deckel des AIO-Gefäßes geschlossen und bei 46 °C für 2 Stunden inkubiert. Anschließend wurden die AIO-Gefäße bei 14.000 Umdrehungen pro Minute für 6 Minuten zentrifugiert, und das Filtrat wurde verworfen. Nach dem Waschen für 20 Minuten bei 48 °C mit 100 µl auf 48 °C vorgewärmten Waschpuffer wurde bei 14.000 rpm für 6 Minuten zentrifugiert. Dann wurde der Filter dem AIO-Gefäß entnommen, auf einen Objektträger gelegt, mit einem Anti-Fading-Reagenz überschichtet und unter einem Epifluoreszenzmikroskop betrachtet. Die rot leuchtenden Zellen entsprechen dem *Nostocoida limicola-*ähnlichen Bakterium aus der Gruppe der grünen, nicht-schwefelhaltigen Bakterien.

### Beispiel 2: Relative Quantifizierung von filamentösen Rhodobacter sphaeroides-ähnlichen Bakterien im Belebtschlamm

### Schritt A: Zellfixierung

Zur Zellfixierung werden 2 ml-Reaktionsgefäße sowie eine Tischzentrifuge (14.000 Umdrehungen/Minute) benötigt.

Zur Paraformaldehyd-Zellfixierung wurden folgende Lösungen verwendet:
- PBS-Stammlösung (NaₓPO₄)
   200 mmol/l NaH₂PO₄
   200 mmol/l Na₂HPO₄ (pH 7,2-7,4)
- 3 x PBS-Lösung
   390 mmol/l NaCl
   30 mmol/l NaₓPO₄ (PBS-Stammlösung) (pH 7,2-7,4)
- 1 x PBS-Lösung
   130 mmol/l NaCl
   10 mmol/1 NaₓPO₄ (PBS-Stammlösung) (pH 7,2-7,4)
- 4 %-ige Paraformaldehydlösung:
   herstellbar durch Zugabe von 3 g Paraformaldehyd zu 30 ml auf 60 °C erhitztem H₂O_{bidest}; anschließend tropfenweise Zugabe von 1 mol/l NaOH bis zum vollständigen Lösen des Paraformaldehyds; anschließend Hinzufügen von 16,6 ml 3 x PBS, Abkühlen der Lösung auf etwa 20 °C; Einstellung des pH-Wertes mit 1 mol/l HCl auf 7,2 - 7,4; Sterilfiltration der fertigen PFA-Lösung über einen 0,2 µm-Filter (MILLIPORE, Eschborn) und Aufbewahrung bei 4 °C für etwa einen Tag.

Die Zellfixierung wurde auf folgende Weise durchgeführt:
1. Zugabe von drei Teilen 4 %-iger Paraformaldehydlösung zu einem Teil frisch gezogener Belebtschlammprobe (z.B. 30 ml 4 %-ige Paraformaldehydlösung zu 10 ml Belebtschlamm).
2. Inkubation für 3 Stunden bei 4 °C.
3. Zentrifugation bei 8000 Umdrehungen pro Minute für 6 Minuten.
4. Verwerfen des Überstandes und vollständige Resuspension des Pellets in 1 ml 1×PBS.
5. Zentrifugation bei 8000 Umdrehungen pro Minute für 6 Minuten.
6. Verwerfen des Überstandes (das Pellet verbleibt im Cap).
7. Resuspension des Pellets mit 250 µl 1xPBS
8. Zugabe von 250 µl-18 °C-kaltem Ethanol
9. Gründliches Wirlen und gegebenenfalls Lagerung bei -18 °C lagern.

Auf diese Weise wurde eine fixierte Belebtschlammprobe erhalten.

### Schritt B: Hybridisierung

Für die in situ-Hybridisierung werden benötigt:
- Tischzentrifuge (14.000 Umdrehungen pro Minute)
- Fluorometer
- 1 Heizblock und 1 Wasserbad, oder 2 Heizblöcke
- Hybridisierungsofen
- All-In-One-Gefäße mit Auffanggefäß
- Formamid (Merck, Darmstadt, Deutschland)
- Sonden-Arbeitslösungen zu je 50 ng/ µl:
- Ablösepuffer: 0,01 mol/l Tris/HCl (pH 9,0)
- Hybridisierungspuffer (2 ml mit 20 % Formamidgehalt):

| | |
|---|---|
| 5 mol/l NaCl | 360 µl |
| 1 mol/l Tris/HCl pH 8,0 | 40 µl |
| Formamid | 400 µl |
| 10 % (m/v) SDS | 2 µl |
| H₂O _{bidest} | 1200 µl |

- Waschpuffer (2 ml):

| | |
|---|---|
| 5 mol/l NaCl | 86 µl |
| 1 mol/l Tris/HCl pH 8,0 | 40 µl |
| 0,5 mol/l EDTA pH 8,0 | 20 µl |
| 10 % (m/v) SDS | 2 µl |
| H₂O _{bidest} | 1850 µl |

### Durchführung:

5 bis 500 µl der fixierten Zellsuspension wurden in die Mitte eines AIO-Gefäßes pipettiert und bei 14.000 Umdrehungen pro Minute für 6 Minuten abzentrifugiert. Das Filtrat wurde verworfen. Das AIO-Gefaß wurde für 5 Minuten bei 80 °C auf einem Heizblock inkubiert. Nach der Zugabe von 20 µl des auf 46 °C vorgewärmten Hybridisierungspuffers wurden 2 µl jeder Oligonukleotidsonde (Konzentration 50 ng/µl) hinzugefügt. Anschließend wurde der Deckel des AIO-Gefäßes geschlossen und bei 46 °C für zwei Stunden inkubiert. Daraufhin wurden die AIO-Gefäße bei 14.000 rpm für 6 Minuten zentrifugiert. Nach dem Waschen für 20 Minuten bei 48 °C mit 100 µl auf 48 °C vorgewärmten Waschpuffer wurde bei 14.000 rpm für 6 Minuten zentrifugiert. Das AIO-Gefäß wurde in ein ungebrauchtes Auffanggefäß überführt, mit 120 µl Ablösepuffer befüllt und 15 Minuten bei 80 °C inkubiert. Anschließend wurde das Auffanggefäß zusammen mit der Filtereinheit bei 14.000 rpm 6 Minuten lang zentrifugiert, die Filtereinheit entfernt und das Filtrat im Dunkeln auf Eis gelagert. Die Fluoreszenz des Filtrates wurde in einem Fluorometer quantifiziert. Die Fluoreszenzmengen, die für die Sonde Rho-645 gemessen wurden, korrespondieren mit der relativen Menge an *Rhodobacter sphaeroides-*ähnlichen filamentösen Bakterien der untersuchten Probe. Diese Mengen wurden mit den Mengen verglichen, die mit der mit einem anderen Farbstoff markierten und in der selben Probe angewandten EUB338-Sonde gemessen werden konnten. Aus dem ermittelten Verhältnis dieser beiden Werte ergibt sich die relative Menge an untersuchten filamentösen *Rhodobacter sphaeroides-*ähnlichen Bakterien.

### Beispiel 3: Nachweis von filamentösen Bakterien der Gruppe der grünen, nicht-schwefelhaltigen Bakterien in Wasserproben

### Schritt A: Zellfixierung

### Laborausstattung:

- All-In-One-Gefäße (AIO-Gefäße)
- Tischzentrifuge (14.000 Umdrehungen pro Minute)

Zur PFA-Zellfixierung verwendete Lösungen:
- Formaldehyd
- 1 x PBS-Lösung
   130 mmol/l NaCl
   10 mmol/l NaₓPO₄ (PBS-Stammlösung) (pH 7,2-7,4)

### Durchführung:

Nach Zugabe von 500 µl Wasserprobe in das AIO-Gefäß wurde bei 14.000 rpm für 5 Minuten abzentrifugiert. Anschließend wurden 100 µl 1% Formaldehyd zugegeben und 30 Minuten lang bei Raumtemperatur inkubiert, gefolgt von einer Zentrifugation bei 14.000 rpm für 5 Minuten. Dann wurden 500 µl 1 x PBS aufgebracht und erneut bei 14.000 rpm 5 Minuten lang zentrifugiert. Nach dem Verwerfen des Zentrifugats wurde das AIO-Gefäß entweder bei -20° C gelagert oder sofort weiterverwendet.

### Schritt B: Hybridisierung

### Laborausstattung:

- Tischzentrifuge (14.000 rpm)
- Fluorometer
- 1 Heizblock und 1 Wasserbad oder alternativ 2 Heizblöcke
- Hybridisierungsofen

### Materialien:

- All-In-One-Gefäße mit Auffanggefäß
- Formamid (Merck, Darmstadt)
- Sonden-Arbeitslösungen zu je 50 ng/µl :
- Ablösepuffer (2 ml):
0,01 mol/l Tris/HCl (pH 9,0)
- Hybridisierungspuffer ( 2 ml mit 35 % Formamidgehalt):

| | |
|---|---|
| 5 mol/l NaCl | 360 µl |
| 1 mol/l Tris/HCl (pH 8,0) | 40 µl |
| Formamid | 700 µl |
| 10 % (m/v) SDS | 2 µl |
| H₂O _{bidest} | 900 µl |

- Waschpuffer (2 ml):

| | |
|---|---|
| 5 mol/l NaCl | 28 µl |
| 1 mol/l Tris/HCl (pH 8,0) | 40 µl |
| 0,5 mol/l EDTA (pH 8,0) | 20 µl |
| 10 % (m/v) SDS | 2 µl |
| H₂O _{bidest} | 1910 µl |

### Durchführung:

Das AIO-Gefäß mit der fixierten Zellsuspension wurde bei 14.000 rpm für 5 Minuten zentrifugiert. Anschließend wird das Filtrat im Auffanggefäß verworfen und auf das Pellet wurden 500 µl einer 50 %-igen Ethanollösung pipettiert und 2 Minuten lang inkubiert. Nach dem erneuten Abzentrifugieren (14.000 rpm, 2 Minuten) wurde das Pellet für 2 Minuten mit 500 µl einer 70 %-igen Ethanollösung inkubiert und erneut einer Zentrifugation unterzogen (14.000 rpm, 2 Minuten). Danach wurden nochmals 500 µl einer 96 %-igen Ethanollösung auf das Pellet pipettiert und 2 Minuten inkubiert. Nach einem anschließenden Zentrifugationsschritt (14.000 rpm, 5 Minuten) wurde das AIO-Gefäß für 5 Minuten bei 80 °C auf einem Heizblock inkubiert. Nach Zugabe von 20 µl des auf 46 °C vorgewärmten Hybridisierungspuffers wurden 2 µl jeder Oligonukleotidsonde (Konzentration 50 ng/µl) hinzugefügt. Anschließend wurde der Deckel des AIO-Gefäßes geschlossen und bei 46 °C 2 Stunden lang inkubiert. Anschließend wurden die AIO-Gefäße bei 14.000 rpm 6 Minuten lang zentrifugiert, und der Überstand wurde verworfen. Nach dem Waschen für 20 Minuten bei 48 °C mit 100 µl auf 48 °C vorgewärmten Waschpuffer wurde bei 14.000 rpm 6 Minuten lang zentrifugiert. Dann wurde der Filter dem AIO-Gefäß entnommen, auf einen Objektträger gelegt, mit einem Anti-Fading Reagenz überschichtet und unter einem Epifluoreszenzmikroskop betrachtet. Die rot leuchtenden Zellen entsprechen den filamentösen Bakterien der Gruppe der grünen, nicht schwefelhaltigen Bakterien.

### Abbildungen:

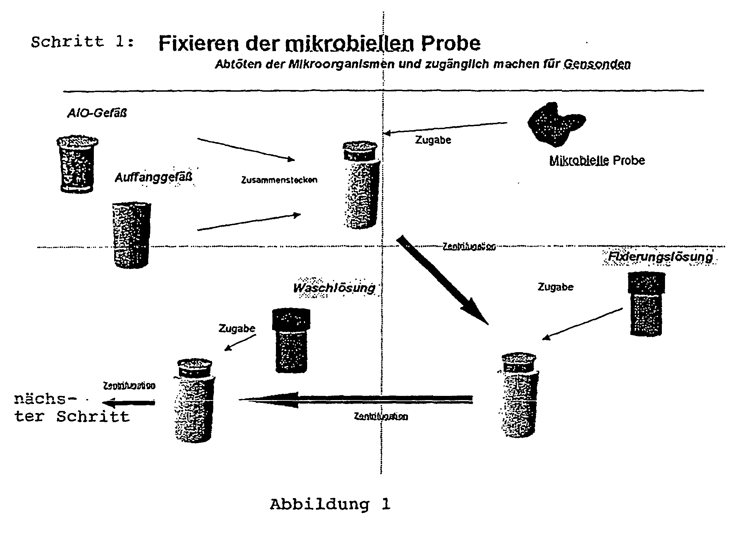

Abbildung 1 zeigt den Schritt a) einer bevorzugten Ausführungsform des erfindungsgemäßen Verfahrens, bei dem die in einer Probe enthaltenen Mikroorganismen auf einem Träger in einem AIO-Gefäß fixiert wurden.

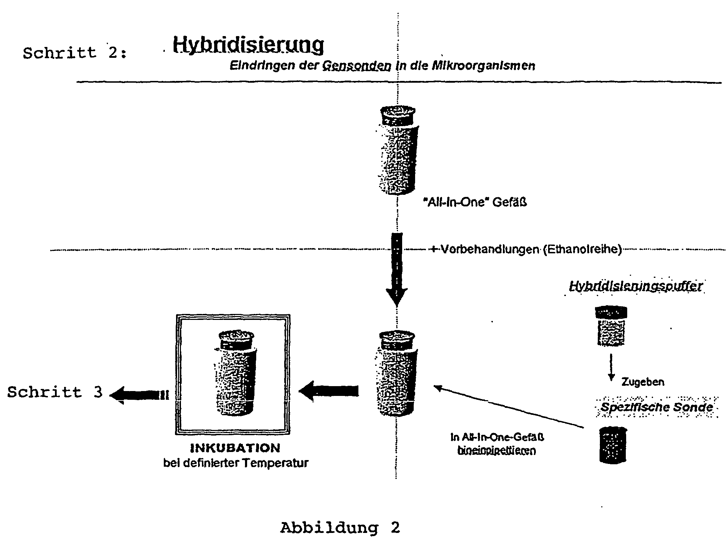

Abbildung 2 zeigt den Schritt b) des erfindungsgemäßen Verfahrens, bei dem die fixierten Mikroorganismen mit nachweisbaren Nukleinsäuresonden auf einem Träger inkubiert werden.

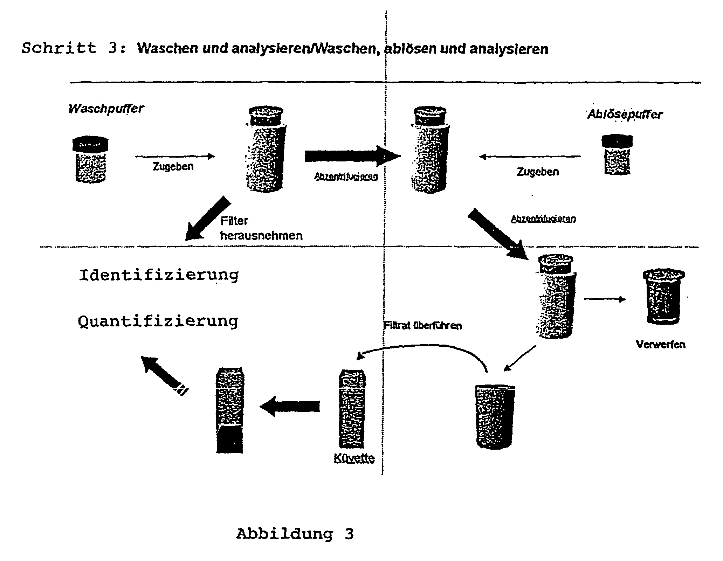

Abbildung 3 zeigt die Schritte c) - e) des erfindungsgemäßen Verfahrens. Nach dem Entfernen der nicht hybridisierten Nukleinsäuresondenmoleküle, können die hybridisierten Nukleinsäuresondenmoleküle nach Herausnahme des Filters identifiziert werden. Alternativ oder zusätzlich können die mittels Zugabe eines Ablösepuffers abgelösten Nukleinsäuresondenmoleküle quantifiziert werden.

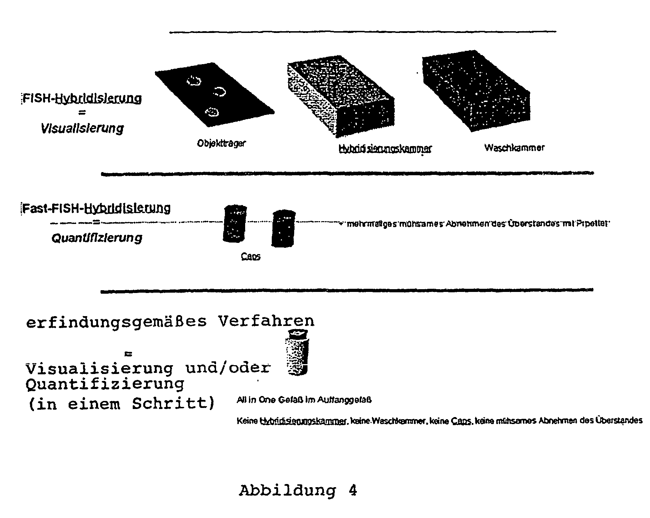

Abbildung 4 zeigt einen Vergleich des AIO(All in One)-Gefäßes als Bestandteil des erfindungsgemäßen Kits sowie des erfindungsgemäßen Verfahrens mit den bekannten Verfahren der FISH- bzw. Fast-FISH-Hybridisierung sowie der dafür benötigten Vorrichtungen.

## Patentansprüche

1. Kit zum Nachweis von Mikroorganismen in einer Probe mittels einer Nukleinsäuresonde, umfassend ein Gefäß (AIO-Gefäß), welches einen Filter enthält, ein Auffanggefäß, in welches das AIO-Gefäß einbringbar ist, mindestens eine Nukleinsäuresonde zum spezifischen Nachweis eines Mikroorganismus sowie gegebenenfalls mindestens eine Nukleinsäuresonde zum Durchführen einer Negativkontrolle, und gegebenenfalls einen Hybridisierungspuffer,
wobei in dem AIO-Gefäß die folgenden Schritte durchführbar sind:
a) Fixieren der in der Probe enthaltenen Mikroorganismen auf dem Filter;
b) Inkubieren der auf dem Filter fixierten Mikroorganismen mit nachweisbaren Nukleinsäuresondenmolekülen;
c) Entfernen nicht hybridisierter Nukleinsäuresondenmoleküle; und
d) Ablösen der hybridisierten Nukleinsäuresondenmoleküle.

2. Kit nach Anspruch 1, wobei der Filter aus regenerierter Cellulose ist.

3. Kit nach Anspruch 1 oder 2, wobei der Filter von dem Gefäß abnehmbar ist.

4. Kit nach einem der vorangehenden Ansprüche, wobei das AIO-Gefäß und/oder das Auffanggefäß verschließbar ist.

5. Kit nach einem der vorangehenden Ansprüche, umfassend mindestens die Nukleinsäuren 5'-TCACGGACTTCAGGCGTT-3' und/oder 5'-TGCGACTTGCATGCATTAGG-3' als spezifische Sonden zum Nachweis von filamentösen Bakterien aus der Gruppe der grünen, nicht-schwefelhaltigen Bakterien, mindestens die Nukleinsäure 5'-ACCAGCCCCTGATACCCT-3' als spezifische Sonde zum Nachweis von *Nostocoida limicola-*ähnlichen Bakterien und/oder mindestens die Nukleinsäure 5'-TCT CGA CCT CAA GAA CAG-3' als spezifische Sonde zum Nachweis von filamentösen *Rhodobacter sphaeroides-*ähnlichen Bakterien.

6. Verfahren zum Nachweis von Mikroorganismen in einer Probe mittels einer Nukleinsäuresonde, umfassend die folgenden Schritte:
a) Fixieren der in der Probe enthaltenen Mikroorganismen auf einem Filter;
b) Inkubieren der auf dem Filter fixierten Mikroorganismen mit nachweisbaren Nukleinsäuresondenmolekülen;
c) Entfernen nicht hybridisierter Nukleinsäuresondenmoleküle, und
d1) Identifizieren der hybridisierten Nukleinsäuresondenmoleküle und/oder
d2) Ablösen und Quantifizieren der abgelösten Nukleinsäuresondenmoleküle,
wobei mindestens die Schritte b) und c) sowie das Ablösen in Schritt d2) in einem Gefäß (AIO-Gefäß), das den Filter enthält, durchgeführt werden und das Gefäß mindestens bei den Schritten c) und d2) in ein Auffanggefäß eingebracht wird.

7. Verfahren nach Anspruch 6, wobei auch Schritt a) in dem AIO-Gefäß durchgeführt wird.

8. Verfahren nach Anspruch 6 oder 7, wobei der Filter aus regenerierter Cellulose ist.

9. Verfahren nach einem der Ansprüche 6 bis 8, wobei der Filter zur Identifizierung der hybridisierten Nukleinsäuresondenmoleküle entnommen wird.

10. Verfahren nach einem der Ansprüche 6 bis 9, wobei bei Durchführung von Schritt d2) die abgelösten Nukleinsäuresondenmoleküle in einem Auffanggefäß gesammelt werden.

11. Verfahren nach einem der Ansprüche 6 bis 10, wobei das Ablösen der hybridisierten Nukleinsäuresondenmoleküle ohne Einsatz von Formamid erfolgt.

12. Verfahren nach einem der Ansprüche 6 bis 11, wobei die in Schritt d2) verwendete Ablöselösung ausgewählt ist aus der Gruppe, bestehend aus Wasser, gepuffertem Wasser, DMSO und SSC.

13. Verfahren nach Anspruch 12, wobei es sich bei der Ablöselösung um 0,001 - 1,0 mol/l Tris/HCl, pH 9,0 +/- 2,0 handelt.

14. Verfahren nach Anspruch 13, wobei es sich bei der Ablöselösung um 0,01 mol/l Tris/HCl, pH 9,0 +/- 2,0 handelt.

15. Verfahren nach einem der Ansprüche 6 bis 14, wobei Schritt d2) bei einer Temperatur von 50-100 °C erfolgt.

16. Verfahren nach Anspruch 15, wobei Schritt d2) bei einer Temperatur von unter 100 °C erfolgt.

17. Verfahren nach Anspruch 16, wobei Schritt d2) bei einer Temperatur von ungefähr 80 °C durchgeführt wird.

18. Verfahren nach einem der Ansprüche 6 bis 17, wobei die Nukleinsäuresonde komplementär zu einer chromosomalen oder episomalen DNA, einer mRNA oder rRNA eines nachzuweisenden Mikroorganismus ist.

19. Verfahren nach einem der Ansprüche 6 bis 18, wobei die Nukleinsäuresonde kovalent mit einem detektierbaren Marker verbunden ist.

20. Verfahren nach Anspruch 19, wobei der detektierbare Marker ausgewählt ist aus der Gruppe der folgenden Marker:
- Fluoreszenzmarker,
- Chemolumineszenzmarker,
- radioaktiver Marker,
- enzymatisch aktive Gruppe,
- Hapten,
- durch Hybridisierung nachweisbare Nukleinsäure.

21. Verfahren nach einem der Ansprüche 6 bis 20, wobei der Mikroorganismus ein einzelliger Mikroorganismus ist.

22. Verfahren nach einem der Ansprüche 6 bis 21, wobei der Mikroorganismus eine Hefe, ein Bakterium, eine Alge oder ein Pilz ist.

23. Verfahren nach Anspruch 22, wobei der Mikroorganismus ein filamentöses Bakterium der Gruppe der grünen, nicht-schwefelhaltigen Bakterien ist, ein *Nostocoida limicola-*ähnliches Bakterium oder ein filamentöses Rhodobacter *sphaeroides-*ähnliches Bakterium ist.

24. Verfahren nach einem der Ansprüche 6 bis 23, wobei die Probe eine Umweltprobe und aus Wasser, Boden oder Luft entnommen ist.

25. Verfahren nach einem der Ansprüche 6 bis 23, wobei die Probe eine Lebensmittelprobe ist.

26. Verfahren nach Anspruch 25, wobei die Probe aus Milch oder Milchprodukten, Trinkwasser, Getränken, Backwaren oder Fleischwaren entnommen ist.

27. Verfahren nach einem der Ansprüche 6 bis 23, wobei die Probe eine medizinische Probe ist.

28. Verfahren nach Anspruch 27, wobei die Probe aus Gewebe, Sekreten oder Stuhl gewonnen ist.

29. Verfahren nach einem der Ansprüche 6 bis 23, wobei die Probe aus Abwasser gewonnen ist.

30. Verfahren nach Anspruch 29, wobei die Probe aus Belebtschlamm, Faulschlamm oder anaerobem Schlamm gewonnen ist.

31. Verfahren nach einem der Ansprüche 6 bis 23, wobei die Probe aus einem Biofilm gewonnen wird.

32. Verfahren nach Anspruch 31, wobei der Biofilm aus einer industriellen Anlage gewonnen wird, bei der Abwasserreinigung gebildet wurde oder ein natürlicher Biofilm ist.

33. Verfahren nach einem der Ansprüche 6 bis 23, wobei die Probe einem pharmazeutischen oder kosmetischen Produkt entnommen ist.

34. Verfahren nach einem der Ansprüche 6 bis 33, wobei das Verfahren automatisierbar im Mikrotiterformat durchgeführt wird.

## Claims

1. A kit for the detection of microorganisms in a sample by means of a nucleic acid probe, which comprises one vessel (AIO vessel) containing a filter, one collecting vessel, into which the AIO vessel is insertable, at least one nucleic acid probe for the specific detection of a microorganism, and optionally, at least one nucleic acid probe for carrying out a negative control, and optionally, one hybridization buffer,
wherein the following steps are performable within the AIO vessel:
a) fixing the microorganisms contained in the sample on the filter,
b) incubating the microorganisms fixed on the filter with detectable nucleic acid probe molecules,
c) removing non-hybridized nucleic acid probe molecules, and
d) separating the hybridized nucleic acid probe molecules.

2. The kit according to claim 1, wherein the filter consists of regenerated cellulose.

3. The kit according to claim 1 or 2, wherein the filter is removable from the vessel.

4. The kit according to any of the preceding claims, wherein the AIO vessel and/or the collecting vessel is closable.

5. The kit according to any of the preceding claims, which comprises at least the nucleic acids 5'-TCACGGACTTCAGGCGTT-3' and/or 5'-TGCGACTTGCATGCATTAGG-3' used as specific probes for the detection of filamentous bacteria from the group of green non-sulfur bacteria, at least the nucleic acid 5'-ACCAGCCCCTGATACCCT-3' used as specific probe for the detection of *Nostocoida limicola-*like bacteria and/or at least the nucleic acid 5'-TCT CGA CCT CAA GAA CAG-3' used as specific probe for the detection of filamentous *Rhodobacter sphaeroides-*like bacteria.

6. A method for the detection of microorganisms in a sample by means of a nucleic acid probe, comprising the following steps:
a) fixing the microorganisms contained in the sample on a filter,
b) incubating the microorganisms fixed on the filter with detectable nucleic acid probe molecules,
c) removing non-hybridized nucleic acid probe molecules, and
d1) identifying the hybridized nucleic acid probe molecules and/or
d2) separating and quantifying the separated nucleic acid probe molecules,
wherein at least steps b) and c) as well as separating in step d2) are carried out in one vessel (AIO vessel) containing the filter, and wherein the vessel is inserted into one collecting vessel during steps c) and d2).

7. The method according to claim 6, wherein also step a) is carried out in the AIO vessel.

8. The method according to claim 6 or 7, wherein the filter consists of regenerated cellulose.

9. The method according to any of the claims 6 to 8, wherein the filter is removed for identifying the hybridized nucleic acid probe molecules.

10. The method according to any of the claims 6 to 9, wherein in performing step d2) the separated nucleic acid probe molecules are collected in one collecting vessel.

11. The method according to any of the claims 6 to 10, wherein the separation of the hybridized nucleic acid probe molecules is carried out without the use of formamide.

12. The method according to any of the claims 6 to 11, wherein the separation solution used in step d2) is selected from the group consisting of water, buffered water, DMSO and SSC.

13. The method according to claim 12, wherein the separation solution is 0.001 to 1.0 mol/l tris/HCl, pH 9.0 +/- 2.0.

14. The method according to claim 13, wherein the separation solution is 0.01 mol/l tris/HCl, pH 9.0 +/- 2.0.

15. The method according to any of the claims 6 to 14, wherein step d2) is carried out at a temperature of from 50 to 100°C.

16. The method according to claim 15, wherein step d2) is carried out at a temperature of below 100°C.

17. The method according to claim 16, wherein step d2) is carried out at a temperature of about 80°C.

18. The method according to any of the claims 6 to 17, wherein the nucleic acid probe is complementary to a chromosomal or episomal DNA, an mRNA or an rRNA of a microorganism to be detected.

19. The method according to any of the claims 6 to 18, wherein the nucleic acid probe is covalently linked to a detectable marker.

20. The method according to claim 19, wherein the detectable marker is selected from the group of the following markers:
- fluorescence marker,
- chemoluminescence marker,
- radioactive marker,
- enzymatically active marker,
- hapten,
- nucleic acid detectable by hybridization.

21. The method according to any of the claims 6 to 20, wherein the microorganism is a single-cell microorganism.

22. The method according to any of the claims 6 to 21, wherein the microorganism is a yeast, a bacterium, an alga or a fungus.

23. The method according to claim 22, wherein the microorganism is a filamentous bacterium from the group of green non-sulfur bacteria, a *Nostocoida limicola-*like bacterium or a filamentous *Rhodobacter sphaeroides-*like bacterium.

24. The method according to any of the claims 6 to 23, wherein the sample is an environmental sample taken from water, soil or air.

25. The method according to any of the claims 6 to 23, wherein the sample is a food sample.

26. The method according to claim 25, wherein the sample is taken from milk or dairy products, drinking water, beverages, bakery products or meat products.

27. The method according to any of the claims 6 to 23, wherein the sample is a medical sample.

28. The method according to claim 27, wherein the sample is obtained from tissue, secreta or faeces.

29. The method according to any of the claims 6 to 23, wherein the sample is obtained from waste water.

30. The method according to claim 29, wherein the sample is obtained from activated sludge, digested sludge or anaerobic sludge.

31. The method according to any of the claims 6 to 23, wherein the sample is obtained from a biofilm.

32. The method according to claim 31, wherein the biofilm is obtained from an industrial plant, is generated in the course of wastewater treatment or is a natural biofilm.

33. The method according to any of the claims 6 to 23, wherein the sample is taken from a pharmaceutical or cosmetic product.

34. The method according to any of the claims 6 to 33, wherein the method is carried out by automatic control in a microtiter format.

## Revendications

1. Kit de détection de micro-organismes dans un spécimen à l'aide d'une sonde d'acide nucléique, comprenant un récipient (récipient AIO), contenant un filtre, un récipient collecteur, dans lequel le récipient AIO est insérable, au moins une sonde d'acide nucléique, pour la détection spécifique d'un micro-organisme et le cas échéant au moins une sonde d'acide nucléique pour la réalisation d'un contrôle négatif et le cas échéant un tampon d'hybridation,
les étapes suivantes étant réalisables dans le récipient AIO :
a) fixation des micro-organismes contenus dans le spécimen sur le filtre ;
b) incubation des micro-organismes fixés sur le filtre avec des molécules détectables sur la sonde d'acide nucléique ;
c) retrait des molécules non hybridées de la sonde d'acide nucléique ; et
d) décrochage des molécules hybridées de la sonde d'acide nucléique.

2. Kit selon la revendication 1, le filtre étant en cellulose régénérée.

3. Kit selon l'une quelconque des revendications 1 ou 2, le filtre pouvant être retiré du récipient.

4. Kit selon l'une quelconque des revendications précédentes, le récipient AIO et/ou le récipient collecteur étant fermables.

5. Kit selon l'une quelconque des revendications précédentes, comprenant au moins les acides nucléiques 5'-TCACGGACTTCAGGCGTT-3' et/ou 5'-TGCGACTTGCATTAGG-3' en tant que sondes spécifiques pour détecter des bactéries filamenteuses du groupe des bactéries vertes non sulfurifères, au moins l'acide nucléique 5'-ACCAGCCCCTGATACCCT-3' en tant que sonde spécifique pour détecter des bactéries similaires aux *Nostocoida limicola* et/ou au moins l'acide nucléique 5'-TCT CGA CCT CAA GAA CAG-3' en tant que sonde spécifique pour détecter des bactéries similaires aux *Rhodobacter sphaeroides* filamenteuses.

6. Procédé de détection de micro-organismes dans un spécimen à l'aide d'une sonde d'acide nucléique, comprenant les étapes suivantes :
a) fixation des micro-organismes contenus dans le spécimen sur un filtre ;
b) incubation des micro-organismes fixés sur le filtre avec des molécules détectables sur la sonde d'acide nucléique ;
c) retrait des molécules non hybridées de la sonde d'acide nucléique ; et
d1) identification des molécules de la sonde d'acide nucléique hybridées et/ou
d2) décrochage et quantification des molécules hybridées de la sonde d'acide nucléique,
au moins les étapes b) et c), ainsi que le décrochage dans l'étape d2) étant réalisés dans un récipient (récipient AIO) contenant un filtre et le récipient étant introduit dans un récipient collecteur, au moins lors des étapes c) et d2).

7. Procédé selon la revendication 6, l'étape a) étant également réalisée dans le récipient AIO.

8. Procédé selon l'une quelconque des revendications 6 ou 7, le filtre étant en cellulose régénérée.

9. Procédé selon l'une quelconque des revendications 6 à 8, le filtre étant retiré pour l'identification des molécules de la sonde d'acide nucléique hybridées.

10. Procédé selon l'une quelconque des revendications 6 à 9, lors de la réalisation de l'étape d2), les molécules décrochées de la sonde d'acide nucléique étant collectées dans un récipient collecteur.

11. Procédé selon l'une quelconque des revendications 6 à 10, le décrochage des molécules hybridées de la sonde d'acide nucléique ayant lieu sans utilisation de formamide.

12. Procédé selon l'une quelconque des revendications 6 à 11, la solution de décrochage utilisée sous l'étape d2) étant choisie dans le groupe constitué par l'eau, l'eau tamponnée, le DMSO et le SSC.

13. Procédé selon la revendication 12, la solution de décrochage étant à 0,001 à 1,0 mole/l de Tris/HCl, pH 9,0 +/-2,0.

14. Procédé selon la revendication 13, la solution de décrochage étant à 0,01 mole/l de Tris/HCI, pH 9,0 +/- 2,0.

15. Procédé selon l'une quelconque des revendications 6 à 14, l'étape d2) étant réalisée à une température comprise entre 50 et 100 °C.

16. Procédé selon la revendication 15, l'étape d2) étant réalisée à une température inférieure à 100 °C.

17. Procédé selon la revendication 16, l'étape d2) étant réalisée à une température approximative de 80 °C.

18. Procédé selon l'une quelconque des revendications 6 à 17, la sonde d'acide nucléique étant complémentaire à un ADN chromosomique ou épisomique, à un ARNm ou à un ARNr d'un micro-organisme devant être détecté.

19. Procédé selon l'une quelconque des revendications 6 à 18, la sonde d'acide nucléique étant covalente avec un marqueur détectable.

20. Procédé selon la revendication 19, le marqueur détectable étant choisi dans le groupe des marqueurs suivants :
- marqueur fluorescent,
- marqueur chimique luminescent,
- marqueur radioactif,
- groupe enzymatique actif,
- haptène
- acide nucléique détectable par hybridation.

21. Procédé selon l'une quelconque des revendications 6 à 20, le micro-organisme étant un micro-organisme monocellulaire.

22. Procédé selon l'une quelconque des revendications 6 à 21, le micro-organisme étant une levure, une bactérie, une algue ou un champignon.

23. Procédé selon la revendication 22, le micro-organisme étant une bactérie filamenteuse du groupe des bactéries vertes non sulfurifères, une bactérie similaire à *Nostocoida limicola,* ou une bactérie similaire à *Rhodobacter sphaeroïdes.*

24. Procédé selon l'une quelconque des revendications 6 à 23, le spécimen étant un échantillon environnemental et prélevé dans de l'eau, le sol ou l'air.

25. Procédé selon l'une quelconque des revendications 6 à 23, le spécimen étant un échantillon de produit alimentaire.

26. Procédé selon la revendication 25, le spécimen étant prélevée dans du lait, des produits lactiques, de l'eau potable, des boissons, des produits de boulangerie ou des produits carnés.

27. Procédé selon l'une quelconque des revendications 6 à 23, le spécimen étant un échantillon médical.

28. Procédé selon la revendication 27, le spécimen étant prélevée dans des tissus, des sécrétions ou des défécations.

29. Procédé selon l'une quelconque des revendications 6 à 23, le spécimen étant prélevée dans des eaux usées.

30. Procédé selon la revendication 29, le spécimen étant prélevée dans des boues activées, des sapropèles ou des boues anaérobies.

31. Procédé selon l'une quelconque des revendications 6 à 23, le spécimen étant prélevée à parti d'un biofilm.

32. Procédé selon la revendication 31, le biofilm étant prélevé dans une installation industrielle, a été formé lors de l'épuration des eaux usées ou est un biofilm naturel.

33. Procédé selon l'une quelconque des revendications 6 à 23, l'spécimen étant un produit pharmaceutique ou cosmétique.

34. Procédé selon l'une quelconque des revendications 6 à 33, le procédé étant réalisé de façon à pouvoir être automatisé dans un format de microtitre.


### SEQUENZPROTOKOLL

<110> Vermicon AG
<120> Verfahren zur Identifizierung, Quantifizierung und Visualisierung von Mikroorganismen
<130> V 7333
<140>
<141>
<150> DE 100 22 304
<151> 2000-05-08
<160> 5
<170> PatentIn Ver. 2.1
<210> 1
<211> 18
<212> DNA
<213> Künstliche Sequenz
<220>
<223> Beschreibung der künstlichen Sequenz: Oligonukelotidsonde
<400> 1
   accagcccct gataccct 18
<210> 2
<211> 18
<212> DNA
<213> Künstliche Sequenz
<220>
<223> Beschreibung der künstlichen Sequenz: Oligonukelotidsonde
<400> 2
   tctcgacctc aagaacag 18
<210> 3
<211> 18
<212> DNA
<213> Künstliche Sequenz
<220>
<223> Beschreibung der künstlichen Sequenz: Oligonukelotidsonde
<400> 3
   gctgcctccc gtaggagt 18
<210> 4
<211> 18
<212> DNA
<213> Künstliche Sequenz
<220>
<223> Beschreibung der künstlichen Sequenz: Oligonukelotidsonde
<400> 4
   tcacggactt caggcgtt 18
<210> 5
<211> 20
<212> DNA
<213> Künstliche Sequenz
<220>
<223> Beschreibung der künstlichen Sequenz: oligonukelotidsonde
<400> 5
   tgcgacttgc atgcattagg 20